# FASCICULE DE BREVET EUROPEEN

(11) **EP 0 646 001 B1**
(45) Date de publication et mention de la délivrance du brevet: **17.09.1997**
(21) Numéro de dépôt: 93913131.4
(22) Date de dépôt: 15.06.1993
(51) Int. Cl.: A61K 9/16

(54) **MICROSPHERES EN POLYMERE BIORESORBABLE, EXEMPTES DE TENSIOACTIF, LEUR PREPARATION ET LEUR APPLICATION COMME MEDICAMENT**
MIKROKÜGELN AUS BIORESORBIERBAREM POLYMER OHNE TENSID, VERFAHREN ZU IHRER HERSTELLUNG UND IHRE VERWENDUNG ALS ARZNEIMITTEL
BIORESORBABLE POLYMER MICROSPHERES FREE FROM SURFACE ACTIVE AGENTS, THEIR PREPARATION AND APPLICATION AS DRUG

(30) Priorité: 15.06.1992 FR 9207186
(43) Date de publication de la demande: 05.04.1995
(73) Titulaire: CENTRE NATIONAL DE LA RECHERCHE SCIENTIFIQUE (CNRS), 75016 Paris (FR)
(72) Inventeur: VERT, Michel, F-34170 Castelnau-le-Lez (FR); COUDANE, Jean, F-34000 Montpellier (FR); USTARIZ, Christine, F-34000 Montpellier (FR); SCHWACH, Grégoire, F-34000 Montpellier (FR)
(74) Mandataire: Tonnellier, Jean-Claude
(86) Numéro de dépôt international: FR9300576
(87) Numéro de publication internationale: WO9325191

(56) Documents cités:
- EP-A- 0 202 065
- WO-A-88/08300
- US-A- 4 818 542
- DATABASE WPI Derwent Publications Ltd., London, GB; AN 88017930 & JP,A,62 280 220 (TAKI KAGAKU KK)

## Description

L'invention a pour objet de nouvelles microsphères en polymère biorésorbable, exemptes d'agent tensioactif, leur préparation et leur application.

L'encapsulation de substances actives dans des matrices polymères, afin de protéger ces substances actives ou de les libérer progressivement, est bien connue.

On connaît notamment divers systèmes, encore appelés systèmes à effet retard, qui libèrent progressivement dans l'organisme où ils sont implantés des principes pharmacologiquement actifs d'intérêt thérapeutique qui sont dispersés dans une matrice polymère. Les polymères utilisés peuvent être dérivés de polymères naturels (cellulose ou protéines), ou peuvent être des polymères synthétiques.

Parmi les matériaux susceptibles d'être implantés dans l'organisme, les polymères biorésorbables, c'est-a-dire les polymères qui se dégradent progressivement dans l'organisme et dont les produits de dégradation sont éliminés par métabolisation ou par excrétion, sont particulièrement intéressants, notamment car ils rendent inutile une intervention chirurgicale destinée à retirer l'implant à l'issue de sa période de fonctionnement. C'est le cas des polyesters dérivés des hydroxyacides. Ces polyesters sont encore appelés, par abréviation, poly(hydroxyacides).

Les systèmes à libération progressive et biorésorbables peuvent aussi se présenter sous la forme de microsphères qui peuvent être administrées par voie orale, intramusculaire ou intraveineuse.

On entend ici par microsphère un système sphérique plein dont le diamètre moyen n'excède pas quelques centaines de micromètres (en particulier 500 µm), cette notion englobant des sphères (parfois appelées nanosphères) de diamètre moyen inférieur au micromètre.

Le principal avantage des microsphères est qu'elles permettent d'administrer facilement par injection des principes actifs ayant une durée d'action prolongée grâce à leur libération progressive, ce qui améliore l'action thérapeutique de ces principes actifs et réduit leur toxicité éventuelle. Cette méthode évite la mise en place d'implants qui peuvent être la source d'inflammations ou d'infections. Elle évite aussi les administrations répétées puisque l'action du principe actif se fait sur une durée plus longue.

Les microsphères ne contenant pas de principe actif ont également une utilité en thérapeutique : elles peuvent servir notamment à provoquer l'embolisation des angiomes.

Les microsphères peuvent être obtenues par diverses techniques, notamment selon la méthode dite par évaporation du solvant. Cette méthode peut être décrite de la façon suivante : le principe actif à encapsuler et le polymère qui constitue les microsphères sont dissous dans un solvant organique volatil non miscible à l'eau. La solution résultante est émulsionnée à l'aide d'un agent tensioactif. L'évaporation progressive du solvant organique conduit à la transformation des gouttelettes de l'émulsion en microsphères solides dans lesquelles le principe actif est emprisonné.

Cette technique fait donc intervenir un agent tensioactif qui a pour fonction de favoriser la stabilité de l'émulsion et donc de garantir la formation correcte des microsphères et la stabilité des suspensions de celles-ci dans les milieux liquides d'injection.

Or, la présence d'un tensioactif à la surface des microsphères, dans un organisme, est de nature à modifier les caractéristiques et les performances du système à effet retard et même de le rendre inexploitable si le tensioactif est toxique, ou s'il est fortement attaché à la surface, car il modifie l'interface organisme/matériau polymère synthétique, C'est en effet cette interface qui conditionne la libération du principe actif et la réponse du milieu d'implantation.

L'un des tensioactifs les mieux adaptés à la fabrication des microparticules à base de polymères est l'alcool polyvinylique (PVA) qui donne lieu à la formation de très peu d'agglomérats de particules et qui permet d'obtenir aisément des microsphères de taille inférieure à 150µm. Cependant, l'utilisation du PVA soulève des problèmes de toxicité potentielle. En effet, ce composé est considéré comme potentiellement cancérigène, en particulier lorsqu'il est administré par voie parentérale.

Ainsi, l'utilisation d'un tensioactif quel qu'il soit, lors de la préparation des microsphères par évaporation du solvant, est susceptible de modifier les caractéristiques du système à effet retard. La morphologie des microsphères (taille, forme, présence de tensioactif à la surface) mais aussi l'efficacité du système aussi bien pour l'incorporation de la substance active que pour le relargage, sont influencées par le tensioactif.

On a maintenant découvert qu'il est possible d'obtenir des microsphères dont les seuls constituants, outre la substance active encapsulée, sont ceux de la matrice polymère, et qui peuvent être préparées sans recourir à l'addition de tensioactifs classiques. En outre, les microsphères obtenues donnent des suspensions stables.

L'invention a donc pour objet des microsphères constituées d'une matrice polymère et éventuellement d'au moins une substance active encapsulée dans ladite matrice polymère réalisée en au moins un polymère ou copolymère d'hydroxyacide, caractérisées par le fait que lesdites microsphères sont exemptes de constituants autres que la substance active (lorsqu'elle est présente) et les constituants du polymère. Autrement dit, les microsphères de l'invention ne contiennent pas de tensioactif,

On a en effet découvert qu'il est possible d'obtenir des microsphères présentant des caractéristiques satisfaisantes, sans utiliser de tensioactifs, en utilisant des mélanges de poly(hydroxyacides), de poids moléculaire élevé et de bas poids moléculaire, comme cela sera précisé ci-après.

On appelle ici poly(hydroxyacide) un polyester dont la structure résulte de l'estérification du groupement hydroxyle d'une molécule d'hydroxyacide par une autre molécule d'hydroxyacide, et ainsi de suite. Il s'agit d'un homopolymère ou d'un copolymère.

Les microsphères de l'invention peuvent être caractérisées à la fois par l'absence d'agents tensioactifs et par une répartition (au moins) bimodale des masses moléculaires des constituants de la matrice polymère.

Les microsphères de l'invention, dont la matrice polymère est constituée essentiellement d'au moins un polymère ou copolymère d'hydroxyacide, peuvent être préparées par un procédé dans lequel on dissout ledit polymère ou copolymère dans une phase organique liquide volatile non miscible à l'eau, on solubilise ou disperse éventuellement une substance active dans ladite phase organique, on prépare une émulsion de la phase organique obtenue dans une phase continue aqueuse, on évapore le solvant et sépare, si désiré, les microsphères solides obtenues de la phase aqueuse, ce procédé étant caractérisé par le fait que ledit polymère ou copolymère comprend au moins un poly(hydroxyacide) de masse moléculaire moyenne au moins égale à 20 000, et au moins un poly(hydroxyacide) de masse moléculaire moyenne inférieure à 5000, qu'après la réalisation de l'émulsion, on ajuste le pH à une valeur de 6-8 par addition d'une base, et que pendant l'étape d'évaporation du solvant organique, on ajuste le pH à une valeur de 6-8 par addition continue ou par additions répétées d'une base.

Comme base, on utilise par exemple l'hydroxyde de sodium ou de potassium ou une amine comme la triéthylamine.

Bien entendu, l'eau peut être remplacée par tout autre liquide dans lequel la phase organique volatile n'est pas soluble.

Parmi les polymères constituant la matrice des microsphères de l'invention, on citera par exemple les poly(alpha-hydroxyacides) les poly(bêta-hydroxyacides) comme par exemple le poly (bêta-hydroxybutyrate), ou les polymères d'acide malique monoestérifié, comme le poly(malate de benzyle) ou des polymères d'autres hydroxyacides, comme la poly(ε-caprolactone), et leurs copolymères. Parmi les polymères préférés on citera plus particulièrement les poly(alpha-hydroxyacides) tels que le poly(acide lactique), le poly(acide glycolique), et leurs copolymères. On sait que ces polymères sont des polyesters linéaires contenant des motifs répétitifs de formule : -[O-CH(R)-CO]-, dans laquelle R représente H dans le cas du poly(acide glycolique) et R représente -CH₃ dans le cas du poly(acide lactique).

On peut également utiliser des copolymères (polyesters) dérivés de ces hydroxyacides, par exemple les copolymères du type poly(acide lactique-co-glycolique) qui contiennent des motifs répétitifs et -[O-CH(CH₃)-CO-]ₚ et [-O-CH₂-CO-]_{q} p et q étant les proportions molaires des motifs lactiques et glycoliques, respectivement.

De préférence, les polymères utilisés sont amorphes, ou au moins partiellement amorphes.

Les poly(hydroxyacides) de masse moléculaire moyenne au moins égale à 20 000, et en particulier au moins égale à 40 000, sont des produits connus ou peuvent être préparés selon les méthodes connues. Certains d'entre eux sont d'ailleurs des produits commerciaux. Par exemple, pour préparer un poly(acide lactique) de masse moléculaire moyenne élevée, ou un copolymère lactique-glycolique, on peut opérer par ouverture de cycle de diesters cycliques (lactide, glycolide ou leurs mélanges) selon les méthodes usuelles. Dans le cas de l'acide lactique, qui existe sous les formes optiquement actives D et L, on utilise de préférence un mélange de ces deux formes contenant par exemple une quantité suffisante de motifs D-lactiques pour que le polymère obtenu soit amorphe. On utilisera notamment un produit de départ constitué d'un mélange de D- et L-lactides choisi de façon que la proportion de motifs D-lactiques soit suffisante pour que le polymère obtenu soit amorphe. Cette proportion peut être facilement déterminée dans chaque cas par de simples expériences de routine. Le plus souvent on utilisera des polymères contenant de 30 à 70 % de motifs dérivés de l'acide D-lactique, et en particulier 50 %.

Les poly(hydroxyacides) de masse moléculaire moyenne faible, inférieure à 5 000, sont également des produits connus, dont certains sont commercialisés, ou peuvent être préparés selon les méthodes connues. Par exemple, pour préparer un poly(acide lactique) de faible masse moléculaire moyenne, on peut opérer par polycondensation de mélanges d'acides L- et D-lactiques, en particulier par polycondensation d'acide D-, L-lactique. Des copolymères correspondants sont préparés de façon analogue.

Les masses moléculaires des polymères utilisés selon l'invention peuvent être déterminées par exemple en solution, par chromatographie par perméation de gel, par comparaison avec des polymères étalons, notamment des étalons de polystyrène.

Les masses moléculaires sont des masses moléculaires en nombre ou en poids. Par exemple, la limite inférieure de 20 000, pour le polymère de masse moléculaire (MM) élevée, est une MM en nombre, et la valeur maximum de 5 000 pour le polymère de faible MM est une MM en poids.

De préférence, on utilise des polymères dont l'indice de polymolécularité n'est pas supérieur à 3 environ, et est en particulier inférieur à 2.

La phase organique utilisée dans le procédé de l'invention comprend principalement un solvant organique, non miscible à l'eau, ou très faiblement soluble dans l'eau, tel que le chloroforme et le dichlorométhane.

Cette phase organique contient à l'état dissous le polymère de masse moléculaire élevée, au moins égale à 20 000, et le polymère de faible masse moléculaire, inférieure à 5000.

Les proportions de polymère de faible masse moléculaire, par rapport au polymère de masse moléculaire élevée, sont les proportions qui permettent d'obtenir des microsphères de qualité suffisante (notamment un état de surface satisfaisant), et ayant les dimensions de particules souhaitées (notamment suffisamment faibles), avec un rendement acceptable. Ces proportions peuvent être déterminées dans chaque cas par de simples expériences de routine. Généralement, on pourra utiliser une proportion de 30 à 300 %, et en particulier de 50 % à 150% en poids de polymère de faible masse moléculaire, par rapport au poids de polymère de masse moléculaire élevée. L'augmentation de la proportion de polymère de faible masse moléculaire permet d'obtenir une diminution de la taille des particules.

La phase organique peut contenir en outre une substance active, qui peut être ajoutée avant ou après l'addition des polymères; la substance active peut être notamment choisie parmi les hormones stéroïdes ou leurs analogues synthétiques (par exemple progestérone, norgestrel, estradiol, noréthistérone, testostérone, hydrocortisone, prednisolone, dexaméthasone), les agents anti-cancéreux (doxorubicine, bléomycine, cis-platine, 5-fluoro-uracile), les antagonistes de narcotiques (par exemple Naltrexone), les neurosédatifs ou les anesthésiques (phénobarbitone, chlorpromazine, méthadone, diazepam), les antibiotiques (érythromycine, gentamycine).

De préférence, la substance active est une substance lipophile, de façon à faciliter son incorporation dans les microsphères.

On réalise ensuite une émulsion en versant progressivement, sous agitation, la phase organique dans une quantité appropriée d'eau. La quantité d'eau peut être déterminée par exemple de façon à utiliser la quantité minimum d'eau, pour limiter la diffusion du principe actif (lorsqu'il est présent) dans la phase aqueuse, la quantité d'eau devant cependant être suffisante pour que la forme des microsphères soit la plus régulière possible. La quantité d'eau peut donc être déterminée dans chaque cas par de simples expériences de routine.

Lorsqu'on désire obtenir des nanoparticules, on peut soumettre l'émulsion à l'action des ultrasons ou d'un homogénéiseur de façon à augmenter l'état de division de l'émulsion. On applique les ultrasons ou homogénéise pendant un temps suffisant pour que les microgouttelettes de l'émulsion atteignent une taille, suffisamment faible, prédéterminée. Ici encore, la mise au point du procédé peut être effectuée par de simples expériences de routine.

Après la réalisation de l'émulsion, on ajuste le pH à une valeur de 6-8, en particulier 7-8, par addition d'une base telle que l'hydroxyde de sodium ou de potassium ou la triméthylamine, car la dissolution partielle des polyacides de faible masse moléculaire confère à l'eau un pH acide. Il est donc important d'ajuster le pH à une valeur suffisante pour que les groupements carboxylique, dans la phase aqueuse ou au contact de celle-ci, soient sous la forme carboxylate qui est nécessaire pour que les microsphères soient stabilisées.

Il n'est pas souhaitable que la phase aqueuse soit amenée à un pH de 7-8 dès le début de la réalisation de l'émulsion, car il faudrait alors redouter que les polymères de faible masse moléculaire ne s'associent pour former des micelles dans la phase aqueuse.

On procède ensuite à l'évaporation du solvant organique, à une température pouvant aller par exemple de 0 à 40 °C. On peut faciliter cette opération en opérant sous pression réduite.

Pendant l'étape d'évaporation du solvant organique, on ajuste le pH à une valeur suffisante, par exemple 6-8, en particulier 7-8, pour les raisons déjà indiquées précédemment, en opérant par addition continue ou par additions répétées d'une base telle que celles mentionnées précédemment.

Bien entendu, pendant l'étape d'évaporation, on maintient une agitation appropriée de l'émulsion.

Après l'étape d'évaporation du solvant organique, on peut recueillir les microsphères, par exemple par centrifugation ou filtration. Elles peuvent ensuite être séchées et éventuellement tamisées.

Les microsphères selon l'invention peuvent être utilisées selon les méthodes connues. En particulier, lorsque la substance active incorporée est un médicament, les microsphères peuvent être utilisées par voie orale, sous forme de poudres, de suspensions aqueuses ou de capsules. Lorsque les microsphères ont une taille suffisament faible, par exemple inférieure à 150 µm, elles peuvent être administrées par injection, selon les méthodes connues. En outre, comme indiqué ci-dessus, des microsphères ne contenant éventuellement pas de substance active peuvent être injectées de façon à provoquer localement une embolisation du système de vascularisation des angiomes.

L'invention a également pour objet un médicament à base de microsphères telles que définies précédemment.

Les exemples suivants illustrent l'invention.

### EXEMPLE 1 :

### A) Préparation des polymères

a) On a préparé un copolymère acide D-, L-lactique/acide glycolique, contenant 25% de motifs dérivés de l'acide glycolique, de la façon suivante :
   On mélange 78,83 g de D,L-lactide et 21,17 g de glycolide pour former 100 g du mélange de monomères polymérisable. On ajoute 0,025 % en poids de trifluorure d'antimoine. Le mélange est dégazé par cyclages répétés de vide et d'azote.Après scellement sous vide, la polymérisation s'effectue en masse à 160°C pendant 3 jours.
   La masse moléculaire en nombre du copolymère, déterminée en chromatographie de perméation de gel, par comparaison à des étalons de polystyrène, est de 38 000. L'indice de polymolécularité (I) est : 1,6.
b) On a préparé un poly(acide lactique) de faible masse moléculaire par polycondensation d'acide D-, L-lactique à 140°C sous vide, pendant trois jours. Le produit réactionnel est refroidi et dissous dans l'acétone. On ajoute de l'eau à la solution de façon à précipiter le polymère, tandis que les monomères résiduels restent en solution. Le polymère est ensuite séché sous vide.
   Sa masse moléculaire en poids, déterminée en chromatographie de perméation de gel, est de 2 500, par rapport à des étalons de polystyrène : I = 1,5.

### B) Préparation des microsphères

On dissout 3,8 g du copolymère et 1,2 g du poly(acide lactique) dans 50 ml de dichlorométhane.

On verse progressivement, en agitant, la solution obtenue dans 1 250 ml d'eau distillée. Le pH, initialement de 5-6, chute rapidement à 3. Au bout d'une minute environ après la fin de l'addition de la phase organique, on ajoute une solution aqueuse de soude 0.2N de façon à ramener le pH à 7,5 environ. On procède ensuite à l'étape d'évaporation du solvant organique, par volatilisation naturelle à température ambiante pendant 4 heures, sous agitation.

En maintenant l'agitation, on ajoute périodiquement, pendant la phase d'évaporation, de la soude 0,2N pour ramener le pH au-dessus de 7. A mesure que le dichlorométhane s'évapore, le pH diminue moins vite et, vers la fin de l'opération, il se stabilise aux environs de 7.

On recueille ensuite par filtration les microsphères obtenues et on les sèche sous pression réduite.

Pour étudier la distribution en taille des microsphères, on utilise 5 tamis ayant des maillages de 500, 250, 125, 100 et 80 µm respectivement. On élimine les particules de taille supérieure à 500 µm. Les quantités de microsphères récupérées dans chaque tamis sont pesées.

Les résultats sont résumés dans le tableau I suivant :

**TABLEAU I**

| Diamètre µm | % en poids |
|---|---|
| entre 250 et 500 | 25,6 |
| entre 125 et 250 | 45,2 |
| entre 100 et 125 | 16,8 |
| entre 80 et 100 | 7,5 |
| < 80 | 4,9 |

En microscopie optique, les microsphères ont une forme sensiblement sphérique et une surface régulière.

Ces résultats ont été confirmés par microscopie électronique à balayage. L'état de surface très régulier des microsphères est comparable à celui des microsphères obtenues en utilisant l'alcool polyvinylique comme agent tensioactif.

### EXEMPLE 2 :

On opère de façon analogue à celle décrite à l'exemple 1,mais en utilisant des parties égales (en poids) du copolymère et du poly(acide lactique) de faible masse moléculaire.

Les microsphères de diamètre inférieur à 500 µm sont obtenues avec un rendement de 61 %. On sépare en outre les particules de dimensions inférieures à 45 µm.

Les résultats sont résumés dans le tableau II suivant :

**TABLEAU II**

| Diamètre µm | % en poids |
|---|---|
| entre 250 et 500 | 6,04 |
| entre 125 et 250 | 5,56 |
| entre 100 et 125 | 7,03 |
| entre 80 et 100 | 27,19 |
| entre 45 et 80 | 48,50 |
| < 45 | 5,68 |

### EXEMPLE 3 :

On dissout 1g de copolymère obtenu à l'exemple 1 (A,a) et 1 g du poly(acide lactique) de faible masse moléculaire obtenu à l'exemple 1 (A,b) ainsi que 0,25 g de progestérone, dans 20 ml de dichlorométhane.

On verse progressivement, en agitant, la solution obtenue dans 500 ml d'eau distillée. Le pH chute rapidement à 3. Au bout d'une minute environ après la fin de l'addition de la phase organique, on ramène le pH à 7,5 par addition de soude 0,2N. On opère ensuite comme décrit à l'exemple 1.

On obtient des microsphères dans lesquelles est incorporée la progestérone.

## Revendications

1. Microsphères constituées d'une matrice polymère et éventuellement d'au moins une substance active encapsulée dans ladite matrice polymère réalisée en au moins un polymère ou copolymère d'hydroxyacide, caractérisées par le fait qu'elles sont exemptes de constituants autres que la substance active éventuellement présente et les constituants de la matrice polymère, et par le fait que la matrice polymère contient au moins un polymère ou copolymère d'hydroxyacide ayant une masse moléculaire supérieure à 20 000, et en particulier supérieure à 40 000, et au moins un polymère ou copolymère d'hydroxyacide ayant une masse moléculaire inférieure à 5 000.

2. Microsphères selon la revendication 1, caractérisées par le fait que le constituant de la matrice polymère a une répartition bimodale des masses moléculaires.

3. Microsphères selon l'une quelconque des revendications précédentes, caractérisées par le fait qu'elles ont des dimensions inférieures à 500 µm.

4. Procédé de préparation de microsphères telles que définies dans l'une quelconque des revendications précédentes, dans lequel on dissout ledit polymère ou copolymère dans une phase organique liquide volatile non miscible à l'eau, on solubilise ou disperse éventuellement une substance active dans ladite phase organique, on prépare une émulsion de la phase organique obtenue dans une phase continue aqueuse, on évapore le solvant et sépare, si désiré, les microsphères solides obtenues de la phase aqueuse, caractérisé par le fait que ladite matrice polymère comprend au moins un hydroxyacide de masse moléculaire moyenne au moins égale à 20 000 et au moins un hydroxyacide de masse moléculaire moyenne inférieure à 5 000, qu'après la réalisation de l'émulsion, on ajuste le pH à une valeur de 6-8 par addition d'une base forte, et que pendant l'étape d'évaporation du solvant organique, on ajuste le pH à une valeur de 6-8 par addition continue ou par additions répétées d'une base.

5. Procédé selon la revendication précédente, caractérisé par le fait que le poly(hydroxyacide) de masse moléculaire moyenne au moins égale à 20 000 est choisi parmi les polymères et copolymères d'alpha-hydroxyacides.

6. Procédé selon la revendication précédente, caractérisé par le fait que lesdits poly(alpha-hydroxyacides) sont choisis parmi les poly(acide lactique) et les poly(acide glycolique).

7. Procédé selon l'une quelconque des revendications 4 à 6, caractérisé par le fait que la proportion de polymère de faible masse moléculaire, par rapport au polymère de masse moléculaire élevée, est suffisante pour obtenir des microsphères ayant des dimensions de particules suffisamment faibles souhaitées.

8. Procédé selon la revendication précédente, caractérisé par le fait que ladite proportion peut varier de 30 à 300 % en poids, et en particulier de 50 à 150 % en poids.

9. Microsphères caractérisées par le fait qu'elles peuvent être obtenues selon le procédé de l'une quelconque des revendications 4 à 8.

10. Médicament à base de microsphères telles que définies dans l'une quelconque des revendications 1 à 3 et 9.

## Claims

1. Microspheres consisting of a polymer matrix and optionally of at least one active substance encapsulated in the said polymer matrix, which matrix is made of at least one hydroxy acid polymer or copolymer, characterized in that the said microspheres are free from constituents other than the active substance which is optionally present and the constituents of the polymer matrix, and in that the polymer matrix contains at least one hydroxy acid polymer or copolymer having a molecular mass of greater than 20,000, and in particular of greater than 40,000, and at least one hydroxy acid polymer or copolymer having a molecular mass of less than 5000.

2. Microspheres according to Claim 1, characterized in that the constituent of the polymer matrix has a bimodal distribution of molecular masses.

3. Microspheres according to any one of the preceding claims, characterized in that they are smaller than 500 µm in size.

4. Process for the preparation of microspheres as defined in any one of the preceding claims, in which the said polymer or copolymer is dissolved in a water-immiscible volatile livid organic phase, an active substance is optionally dissolved or dispersed in the said organic phase, an emulsion of the organic phase obtained is prepared in an aqueous continuous phase, the solvent is evaporated off and, if so desired, the solid microspheres obtained are separated from the aqueous phase, this process being characterized in that the said polymer matrix comprises at least one hydroxy acid with an average molecular mass at least equal to 20,000, and at least one hydroxy acid with an average molecular mass of less than 5000, in that after production of the emulsion, the pH is adjusted to a value of 6-8 by addition of a base, and in that during the step of evaporating the organic solvent, the pH is adjusted to a value of 6-8 by continuous addition or by repeated additions of a base.

5. Process according to the preceding claim, characterized in that the poly(hydroxy acid) with an average molecular mass at least equal to 20,000 is chosen from polymers and copolymers of alpha-hydroxy acids.

6. Process according to the preceding claim, characterized in that the said poly(alpha-hydroxy acid)s are chosen from poly(lactic acid)s and poly(glycolic acid)s.

7. Process according to any one of Claims 4 to 6, characterized in that the proportion of polymer of low molecular mass, relative to the polymer of high molecular mass, is sufficient to obtain microspheres having desired sufficiently low particle sizes.

8. Process according to the preceding claim, characterized in that the said proportion may range from 30 to 300% by weight, and in particular from 50 to 150% by weight.

9. Microspheres characterized in that they may be obtained according to the process of any one of claims 4 to 8.

10. Drug based on microspheres as defined in any one of Claims 1 to 3 and 9.

## Patentansprüche

1. Mikrokügelchen, gebildet aus einer polymeren Matrix und gegebenenfalls mindestens einem Wirkstoff, der in der polymeren Matrix, die aus mindestens einem Hydroxysäure-Polymeren oder -Copolymeren gebildet ist, eingekapselt ist, dadurch gekennzeichnet, daß sie, abgesehen von dem gegebenenfalls vorhandenen Wirkstoff und den Bestandteilen der polymeren Matrix, frei von anderen Bestandteilen sind und daß die polymere Matrix mindestens ein Hydroxysäure-Polymeres oder -Copolymeres mit einer Molekülmasse von mehr als 20000 und insbesondere von mehr als 40000 und mindestens ein Hydroxysäure-Polymeres oder -Copolymeres mit einer Molekülmasse unter 5000 enthält.

2. Mikrokügelchen nach Anspruch 1, dadurch gekennzeichnet, daß der Bestandteil der polymeren Matrix eine bimodale Verteilung der Molekülmassen aufweist.

3. Mikrokügelchen nach einem der vorstehenden Ansprüche, dadurch gekennzeichnet, daß sie Abmessungen unter 500 µm aufweisen.

4. Verfahren zur Herstellung von Mikrokügelchen gemäß der Definition in einem der vorstehenden Ansprüche, bei dem man das Polymere oder Copolymere in einer mit Wasser nichtmischbaren flüchtigen flüssigen organischen Phase löst, in dieser organischen Phase gegebenenfalls einen Wirkstoff löst oder dispergiert, eine Emulsion der erhaltenen organischen Phase in einer kontinuierlichen wäßrigen Phase herstellt, das Lösungsmittel abdampft und gegebenenfalls die erhaltenen festen Mikrokügelchen von der wäßrigen Phase abtrennt, dadurch gekennzeichnet, daß die polymere Matrix mindestens eine Hydroxysäure mit einer durchschnittlichen Molekülmasse von mindestens 20000 und mindestens eine Hydroxysäure mit einer durchschnittlichen Molekülmasse unter 5000 umfaßt, daß man nach der Herstellung der Emulsion den pH-Wert durch Zugabe einer starken Base auf 6-8 einstellt und daß man während der Stufe des Abdampfens des organischen Lösungsmittels den pH-Wert durch kontinuierliche Zugabe oder durch wiederholte Zugabe einer Base auf 6-8 einstellt.

5. Verfahren nach dem vorstehenden Anspruch, dadurch gekennzeichnet, daß die Poly-(hydroxysäure) mit einer durchschnittlichen Molekülmasse von mindestens 20000 unter den Polymeren und Copolymeren von α-Hydroxysäuren ausgewählt wird.

6. Verfahren nach dem vorstehenden Anspruch, dadurch gekennzeichnet, daß die Poly-(alpha-hydroxysäuren) unter Poly-(milchsäure) und Poly-(glycolsäure) ausgewählt werden.

7. Verfahren nach einem der Ansprüche 4-6, dadurch gekennzeichnet, daß der Anteil des Polymeren von geringer Molekülmasse im Verhältnis zum Polymeren von erhöhter Molekülmasse ausreicht, um Mikrokügelchen mit den gewünschten, ausreichend geringen Teilchenabmessungen zu erhalten.

8. Verfahren nach dem vorstehenden Anspruch, dadurch gekennzeichnet, daß dieser Anteil von 30-300 Gew.-% und insbesondere von 50-150 Gew.-% variieren kann.

9. Mikrokügelchen, dadurch gekennzeichnet, daß sie gemäß dem Verfahren nach einem der Ansprüche 4-8 erhältlich sind.

10. Arzneimittel auf der Basis von Mikrokügelchen gemäß der Definition in einem der Ansprüche 1-3 und 9.
